# EUROPEAN PATENT APPLICATION

(11) **EP 3 427 639 A1**
(43) Date of publication of application: **16.01.2019**
(21) Application number: 17181091.4
(22) Date of filing: 13.07.2017
(51) Int. Cl.: A61B 5/00, A61B 5/087

(54) **A WEARABLE SPIROMETRY ARRANGEMENT**

(71) Applicant: Wind Tales BV, 5963 AB Hegelsom (NL)
(72) Inventor: Vredeveldt, Niel Franciscus Josephus, 5963 AB Hegelso (NL)
(74) Representative: Renkema, Jaap

(57) **Abstract**

The invention relates to a wearable spirometry arrangement (1) comprising a spirometer (2) having an inlet (3) for receiving a volume of air inspired and expired by a user (4), and an adjustable support structure (5) having a first end part (6) that is connected with the spirometer, said support structure being configurable to have at least two contact areas with at least one of a torso (10) and a neck (17) of the user, wherein a first contact area (7) of the at least two contact areas is arrangeable on a side of at least one of the torso and the neck of the user that is opposite to a side of at least one of the torso and the neck of the user on which a second contact area (8) of the at least two contact areas is arrangeable.

## Description

### FIELD OF THE INVENTION

The present invention relates to a wearable spirometry arrangement.

### BACKGROUND OF THE INVENTION

Spirometry arrangements are well known in the art and comprise at least one of handheld spirometers, desk top device spirometers and PC-based spirometers that are mounted on a fixed tripod or a trolley. Such spirometry arrangements are not very convenient as a user typically needs to hold the spirometer in at least one of his hands while performing breathing exercises, lung function measurements or lung training. In the case of a PC-based spirometry arrangement, the user is bound to the location of the arrangement, typically a hospital. The skilled person will appreciate that the user can be male or female. For the sake of convenience only, in the remainder of this application the user will be referred to as a male.

Known spirometry arrangements for performing metabolic measurements are conventionally equipped with a cap that is attached to the head of a user for covering at least one of the mouth and the nose of the user. This cap is connected with equipment that measures and analyses air inspired an expired by the user. Such spirometry arrangements are quite inconvenient as the user cannot turn away his mouth from the cap when, for example, he wants to clear his mouth from mucus out of his lungs that arrived in his mouth via his respiratory tract. As spirometry arrangements for performing metabolic measurements usually are quite complex and bulky, a user is bound to the place where such arrangements are located, typically a hospital.

### SUMMARY OF THE INVENTION

It is an object of the present invention to provide a wearable spirometry arrangement that pre-empts or at least reduces at least one of the abovementioned and/or other disadvantages associated with spirometry arrangements known in the art.

Aspects of the present invention are set out in the accompanying independent and dependent claims. Features from the dependent claims may be combined with features from the independent claim as appropriate and not merely as explicitly set out in the claims.

At least one of the abovementioned objects is achieved by a wearable spirometry arrangement comprising a spirometer having an inlet for receiving a volume of air inspired and expired by a user, and an adjustable support structure having a first end part that is connected with the spirometer, the support structure being configurable to have at least two contact areas with at least one of a torso and a neck of the user, wherein a first contact area of the at least two contact areas is arrangeable on a side of at least one of the torso and the neck of the user that is opposite to a side of at least one of the torso and the neck of the user on which a second contact area of the at least two contact areas is arrangeable. In this way, a wearable spirometry arrangement is provided that can be positioned stably on the torso of the user. As a result, the user does not need to use his hands to at least one of position and hold the spirometer while performing breathing exercises, lung function measurements or lung training. This allows the user to perform the breathing exercises, lung function measurements or lung training more freely and less consciously. As a result, user experience can be improved as the user has a more free feeling while doing the aforementioned activities.

Moreover, as the user does not need to use his hands to position and/or hold the spirometer, he can use them for performing other tasks such as interacting with a game console, a tablet or a smartphone to play a game while doing the breathing exercises, lung function measurements or lung training. This provides a way to improve user experience and consequently to increase the user's motivation and endurance to perform and complete the breathing exercises, lung function measurements or lung training.

In addition, as the spirometer is not connected with the head of the user, the user can easily turn away his mouth from the spirometer when he, for example, wants to clear his mouth from mucus. After clearing his mouth the user can easily take the inlet of the spirometer in his mouth again as it remains stably positioned in front of his head upon letting it go. This significantly improves user comfort over existing spirometry arrangements that are connected with the head of the user.

The skilled person will appreciate that the support structure can have any construction that is suitable for enabling the at least two contact areas with at least one of the torso and the neck of the user.

Furthermore, as the wearable spirometry arrangement is lightweight it improves user comfort as the user can perform breathing exercises, lung function measurements or lung training at any desired location, e.g. at home, while traveling, on a holiday location and in a hospital.

Based on the above, the skilled person will appreciate that the wearable hands free spirometry arrangement according to the invention significantly improves both user experience and comfort.

In an embodiment of the wearable spirometry arrangement according to the invention, the adjustable support structure is at least partially deformable. The skilled person will appreciate that deformable is to be construed as deformable by the user by hand. This allows the user to shape the adjustable support structure to for example fit to at least a part of at least one of his torso and his neck. Furthermore, this allows the user to adjust the shape of the adjustable support structure even further when required. Although the adjustable support structure is deformable by hand, it has sufficient stiffness to warrant that its shape is maintained while the user performs the breathing exercises, lung function measurements or lung training and that it remains stably positioned on the user.

In an embodiment of the wearable spirometry arrangement according to the invention, the adjustable support structure is configurable at least over one shoulder of the user. The skilled person will appreciate that the adjustable support structure can also be configured over both shoulders and a part of the back of the user or over both shoulders and the neck of the user.

In an embodiment of the wearable spirometry arrangement according to the invention, the adjustable support structure is provided with at least one support element. In this way it is possible to position the adjustable support structure even more stably.

In an embodiment of the wearable spirometry arrangement according to the invention, the at least one support element has an anatomical shape. In this way the user comfort while wearing the spirometry arrangement can further be improved. The skilled person will appreciate that the anatomical shape of the at least one support element depends on the specific application location on at least one of the torso and the neck of the user.

In an embodiment of the wearable spirometry arrangement according to the invention, the at least one support element comprises at least one soft pad that is arrangeable in abutting contact with at least one of the torso and the neck of the user. In this way the user comfort can even further be improved.

In an embodiment of the wearable spirometry arrangement according to the invention, the adjustable support structure is provided with a control unit that is configured and arranged to communicate with the spirometer. The control unit can be used to at least one of receive, store and analyze data from the spirometer.

In an embodiment of the wearable spirometry arrangement according to the invention, the control unit is accommodated within the at least one support element. By integrating the control unit into the at least one support element, the wearability of the spirometry arrangement can be improved.

In an embodiment of the wearable spirometry arrangement according to the invention, at least one of the spirometer and the control unit is configured and arranged to communicate with a remote system. In this way it is possible to at least one of send and receive data between the remote system and at least one of the spirometer and the control unit. At least one of the spirometer and the control unit can be provided with wireless communication facilities such as for example WiFi and Bluetooth. The skilled person will appreciate that the remote system can be any stationary or mobile electronic system including for example a data analyzing system, a data storage system, a game console, a smartphone and a tablet.

In an embodiment of the wearable spirometry arrangement according to the invention, the adjustable support structure comprises electrical wiring that is arranged between the spirometer and the control unit. The skilled person will appreciate that the electrical wiring can be used to at least one of provide power to the spirometer and the control unit and to enable communication between the spirometer and the control unit.

In an embodiment of the wearable spirometry arrangement according to the invention, the adjustable support structure comprises at least one flexible tubular element. The skilled person will appreciate that it is possible that the entire adjustable support structure is deformable and is implemented as one flexible elongated tubular element. In this way the user can deform the adjustable support structure by hand to shape the adjustable support structure in such a way that it conforms with the anatomical shape of at least a part of the torso and/or the neck of the user with which the adjustable support structure can be placed in an abutting contact. As a result, the user comfort while wearing the spirometry arrangement can be improved even further.

In an embodiment of the wearable spirometry arrangement according to the invention, the adjustable support structure further comprises at least one rigid tubular element that is connected with the at least one flexible tubular element, wherein the at least one flexible tubular element or the at least one rigid tubular element is connected with the spirometer. The skilled person will appreciate that the adjustable support structure can comprise multiple flexible tubular elements that can be arranged in groups that are separated by rigid tubular elements. The rigid tubular elements can have a predetermined anatomical shape. Furthermore, at least one of the flexible and rigid tubular elements can have an elongated shape.

In an embodiment of the wearable spirometry arrangement according to the invention, the adjustable support structure comprises multiple flexible tubular elements and multiple rigid tubular elements that are alternatingly arranged.

In an embodiment of the wearable spirometry arrangement according to the invention, the adjustable support structure has an outer surface that is provided with at least one cover layer. In this way the adjustable support structure can be protected from the environment in which the wearable spirometry arrangement is used. Furthermore, the skilled person will appreciate that depending on the requirements of a specific application of the wearable spirometry arrangement also multiple coaxially arranged cover layers can be applied.

In an embodiment of the wearable spirometry arrangement according to the invention, the at least one cover layer is a resilient layer. In this way, the user comfort while wearing the spirometry arrangement can be improved. When multiple coaxially arranged cover layers are applied, at least the outermost cover layer preferably is a resilient layer. The skilled person will appreciate that the resilient layer can comprise any kind of rubber, a resilient polymer and a solid foam.

### BRIEF DESCRIPTION OF THE DRAWINGS

Further features and advantages of the invention will become apparent from the description of the invention by way of exemplary and non-limiting embodiments of a wearable spirometry arrangement according to the invention.

The person skilled in the art will appreciate that the described embodiments of the wearable spirometry arrangement are exemplary in nature only and not to be construed as limiting the scope of protection in any way. The person skilled in the art will realize that alternatives and equivalent embodiments of the wearable spirometry arrangement can be conceived and reduced to practice without departing from the scope of protection of the present invention.

Reference will be made to the figures on the accompanying drawing sheets. The figures are schematic in nature and therefore not necessarily drawn to scale. Furthermore, equal reference numerals denote equal or similar parts. On the attached drawing sheets,
figure 1 a shows a perspective view of a first exemplary, non-limiting embodiment of a wearable spirometry arrangement according to the invention according to which the adjustable support structure has two contact areas with the torso and the neck of a user respectively;
figure 1b shows a perspective view of the first exemplary, non-limiting embodiment of the wearable spirometry arrangement shown in figure 1 a from a different viewing angle;
figure 2 shows a perspective view of a second exemplary, non-limiting embodiment of a wearable spirometry arrangement according to the invention according to which the adjustable support structure has three contact areas with the torso of a user;
figure 3 shows a perspective view of a third exemplary, non-limiting embodiment of a wearable spirometry arrangement according to the invention according to which the adjustable support structure has four contact areas with the torso of a user;
figure 4 shows a perspective view of a fourth exemplary, non-limiting embodiment of a wearable spirometry arrangement according to the invention wherein the adjustable support structure is at least partially deformable and is provided with one support element that in use can be arranged in abutting contact with at least one of the torso and the neck of the user;
figure 5 shows a perspective view of a fifth exemplary, non-limiting embodiment of the wearable spirometry arrangement according to the invention wherein a control unit is accommodated in the support element of the fourth embodiment as shown in figure 4;
figure 6 shows a perspective view of a sixth exemplary, non-limiting embodiment of the wearable spirometry arrangement according to the invention wherein the adjustable support structure is at least partially deformable and is provided with a control unit that is accommodated in a support element that in use can be arranged in abutting contact with a shoulder of the user; and
figure 7 shows a part of the adjustable support structure according to a first exemplary, non-limiting embodiment thereof.

### DETAILED DESCRIPTION OF EMBODIMENTS

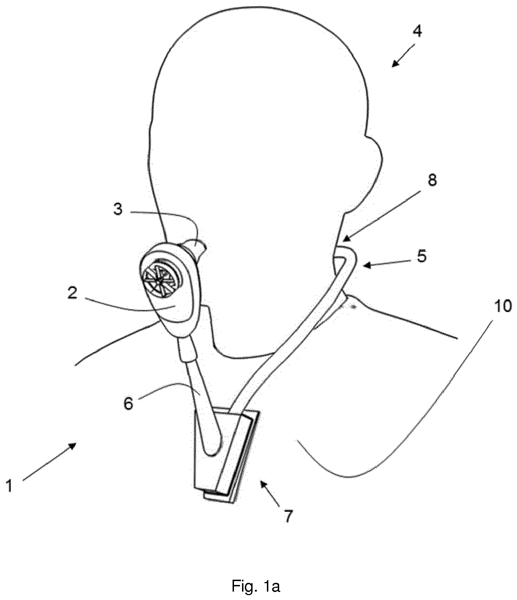

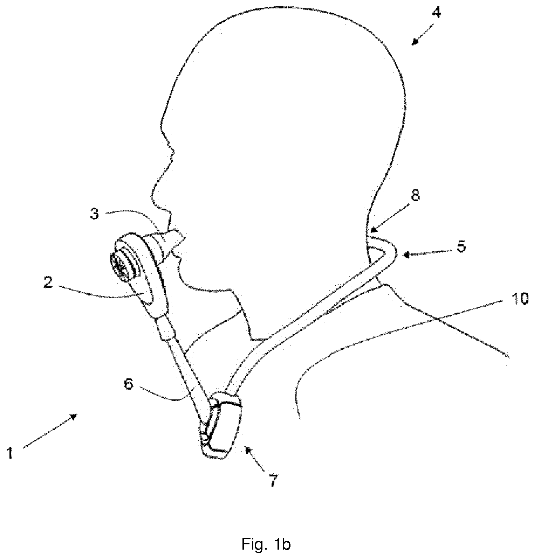

Figure 1 a shows a perspective view of a first exemplary, non-limiting embodiment of a wearable spirometry arrangement 1 according to the invention comprising a spirometer 2 that has an inlet 3 for receiving a volume of air inspired and expired by a user 4. The wearable spirometry arrangement 1 comprises an adjustable support structure 5 that has a first end part 6 that is connected with the spirometer 2. The support structure 5 has two contact areas 7, 8. A first contact area 7 is arranged on the torso 10 of the user 4 and a second contact area 8 is arranged on the neck 17 of the user 4. The first contact area 7 and the second contact area 8 are arranged on opposing sides of the torso and the neck respectively. In this way it is possible to stably position the adjustable support arrangement 5 and consequently the spirometer 2 in front of the head of the user 4. As a result, the user 4 does not need to use his hands to at least one of position and hold the spirometer 2 while performing breathing exercises, lung function measurements or lung training. Instead he can use at least one of his hands to perform other tasks such as interacting with a game console, a tablet or a smartphone to play a game while doing the breathing exercises, lung function measurements or lung training. This provides a way to improve user experience and consequently to increase the user's motivation and endurance to perform and complete the breathing exercises, lung function measurements or lung training. In addition, as the spirometer 2 is not connected with the head of the user 4, the user can easily turn away his mouth from the spirometer 2 when he, for example, want to clear his mouth from mucus. After clearing his mouth the user 4 can easily take the inlet 3 of the spirometer 2 in his mouth again as it remains stably positioned in front of his head upon letting it go. This significantly improves user comfort over existing spirometry arrangements that are connected with the head of the user 4.

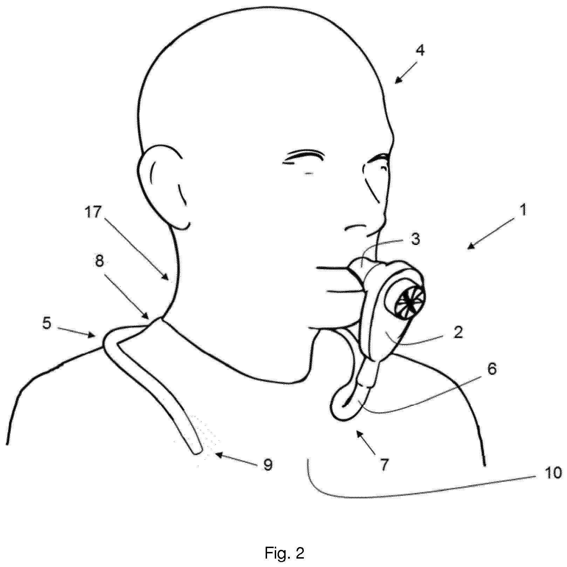

The adjustable support structure 5 shown in figure 1 a is implemented as a flexible and deformable tubular element that is provided with a rigid element that is used to establish a first contact area 7 with the chest of the torso 10. Depending on the specific anatomical dimensions of the user, the length of the first end part 6 can be adjusted by choosing a suitable position of the rigid element along the flexible and deformable tubular element of the adjustable support structure 5. The rigid element in this embodiment has a first non-limiting polygonal shape and comprises a contact element that is arranged in abutting contact with the chest of the torso 10 of the user 4. The flexible first end part 6 can be deformed such that the spirometer 2 can be positioned before the head of the user in such a way that the user can easily interact with the inlet 3 without having to use at least one of his hands. The flexible tubular element can be deformed such that the first contact area 7 and the second contact area 8 can be arranged in abutting contact with respectively the chest of the torso 10 and the neck of the user 4. The skilled person will appreciate that the adjustable support structure 5 can have any construction that is suitable for enabling at least two contact areas 7, 8 with the chest of the torso 10 and the neck of the user 4 as described above. Non-limiting examples of exemplary embodiments of the adjustable support structure 5 will be shown in more detail in figures 2 to 6.

Furthermore, as the wearable spirometry arrangement 1 is lightweight, it improves user comfort as the user 4 can perform breathing exercises, lung function measurements or lung training at any desired location, e.g. at home, while traveling, on a holiday location and in a hospital.

In the embodiment of the wearable spirometry arrangement 1 shown in figure 1 a the adjustable support structure 5 is arranged to be in abutting contact with the chest of the torso 10 and the neck of the user 4 without contacting the shoulder. This can more clearly be observed in figure 1b which provides a perspective view of the first exemplary, non-limiting embodiment of the wearable spirometry arrangement 1 from a different viewing angle. From figure 1b it is clear that the rigid element that is arranged to have a first contact area 7 with the chest of the torso 10 of the user 4 has a shape that is different from the shape of the rigid element shown in figure 1 a. The skilled person will appreciate that the shape of the rigid element is a design parameter that has no significant influence on the performance of the wearable spirometry arrangement 1 other than that it should not impede user experience by being inconvenient.

Moreover, the skilled person will appreciate that it is also possible to arrange the adjustable support structure 5 in many other suitable ways, for example over both shoulders and a part of the back of the user as is shown for example in figure 2.

Figure 2 shows a perspective view of a second exemplary, non-limiting embodiment of a wearable spirometry arrangement 1 according to the invention. The wearable spirometry arrangement 1 comprises an adjustable support structure 5 that has a first end part 6 that is connected with the spirometer 2. The adjustable support structure 5 is implemented as one flexible elongated tubular element that is deformed to obtain a shape that conforms with the anatomical shape of at least a part of the torso 10 of the user 4 with which it is placed in an abutting contact. The deformable support structure 5 has three contact areas 7, 8, 9 with the torso 10 of the user 4. The first contact area 7 and the third contact area 9 are arranged on a side of the torso 10, i.e. the chest, that is opposite to a side of the torso 10 of the user 4, i.e. the back, on which the second contact area 8 is arranged. The skilled person will appreciate that it is also possible to arrange the first contact area 7 and the third contact area 9 on the back of the user 4 and the second contact area 8 on the chest of the user. By arranging the three contact areas as described above, it is possible to stably position the deformable support arrangement 5. Consequently, the spirometer 2 is stably positioned in front of the head of the user 4. The skilled person will appreciate that the deformable support structure 5 can have any construction that is suitable for enabling the three contact areas 7, 8, 9 with the torso 10 of the user 4 as described above.

The deformable support structure 5 as shown in figure 2 is open at the side of the throat of the user 4. The skilled person will appreciate that it is also possible that the deformable support structure 5 for example is closed at the side of the throat and opened at some other location or that the at least partially deformable support structure 5 is completely closed.

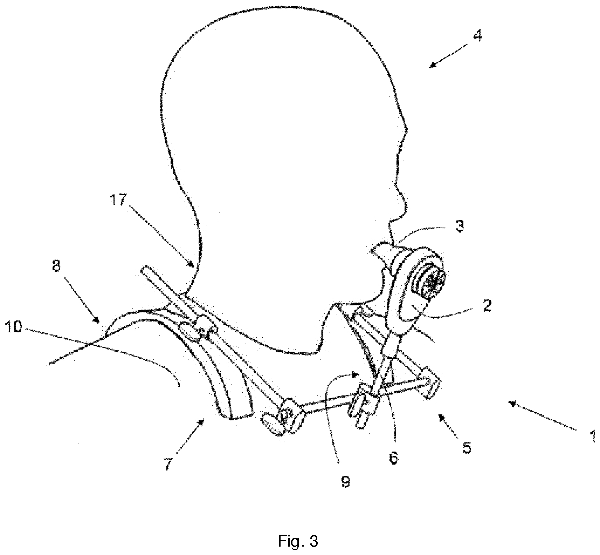

Figure 3 shows a perspective view of a third exemplary, non-limiting embodiment of a wearable spirometry arrangement 1 according to the invention according to which the adjustable support structure 5 has four contact areas with the torso 10 of the user 4. The adjustable support structure 5 is partially deformable as it comprises parts that have been deformed to obtain a shape that conforms with the anatomical shape of at least a part of the torso 10 of the user 4 with which it is placed in an abutting contact. The deformed parts of the adjustable support structure 5 have four contact areas with the torso 10 of the user 4. The skilled person will appreciate that due to the viewing angle only three contact areas of the four contact areas can be seen in figure 3. The first contact area 7 and the third contact area 9 are arranged on a side of the torso 10, i.e. the chest, that is opposite to a side of the torso 10 of the user 4, i.e. the back, on which the second contact area 8 and the not shown fourth contact area are arranged. By arranging the four contact areas as described above, it is possible to stably position the adjustable support arrangement 5. Consequently, the spirometer 2 is stably positioned in front of the head of the user 4.

Furthermore, from figure 3 it will be clear to the skilled person how depending on the specific anatomical dimensions of the user 4, the spirometer 2 can conveniently be positioned before the head of the user by adjusting at least one of the length of the first end part 6 and the length of the other tubular parts of the adjustable support arrangement 5.

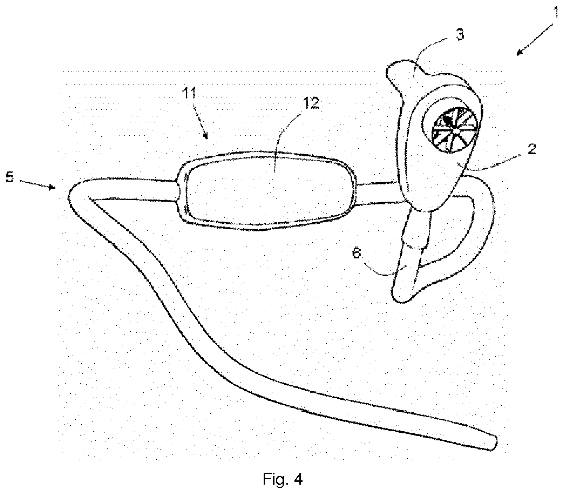

Figure 4 shows a perspective view of a fourth exemplary, non-limiting embodiment of a wearable spirometry arrangement 1 according to the invention wherein the adjustable support structure 5 is at least partially deformable and is provided with one support element 11 that in use can be arranged in abutting contact with at least one of the torso and the neck of the user. In this way it is possible to even more stably position the at least partially deformable support structure 5. The support element 11 shown in figure 4 comprises a soft pad 12 or pouch that is arrangeable in abutting contact with at least one of the torso and the neck of the user. In this way the user comfort can even further be improved. The skilled person will appreciate that the support element 11 can also comprise multiple soft pads that can be arranged in any advantageous configuration for improving the user comfort. The skilled person will also appreciate that the support element 11 can also comprise a hard pad. The support element 11 has an anatomical shape to further improve the user comfort while wearing the spirometry arrangement 1. The skilled person will appreciate that the anatomical shape of the support element 11 depends on the specific application location on the torso and/or the neck of the user.

Furthermore, the skilled person will appreciate that the support element 11 can also be used to accommodate a rechargeable battery pack to provide the spirometer 2 with electrical power.

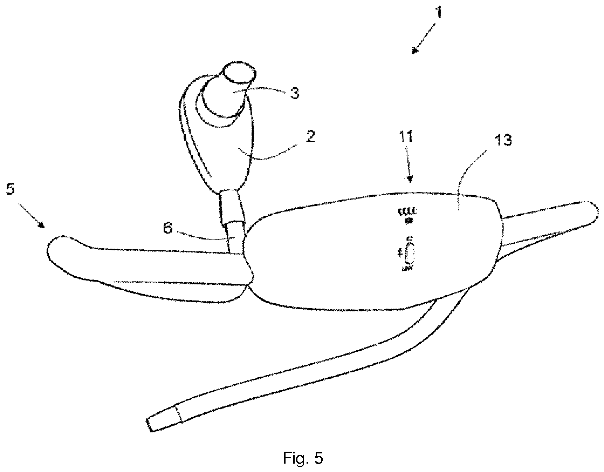

Figure 5 shows a perspective view of a fifth exemplary, non-limiting embodiment of the wearable spirometry arrangement 1 according to the invention wherein a control unit 13 is accommodated in the support element 11 of the fourth embodiment shown in figure 4. By integrating the control unit 13 into the support element 11, the wearability of the spirometry arrangement 1 can be improved. The control unit 13 is configured and arranged to communicate with the spirometer 2. The control unit 13 can be used to at least one of receive, store and analyze data from the spirometer 2. The skilled person will appreciate that the control unit can also be accommodated within the spirometer 2 and that the support element 11 can be used to accommodate a rechargeable battery pack to provide the spirometer 2 and the control unit 13 with electrical power. Moreover, the control unit 13 can be configured and arranged to communicate with a remote system. In this way it is possible to at least one of send and receive data between the control unit 13 and the remote system. The skilled person will appreciate that the remote system can be any stationary or mobile electronic system including for example a data analyzing system, a data storage system, a game console, a smartphone and a tablet.

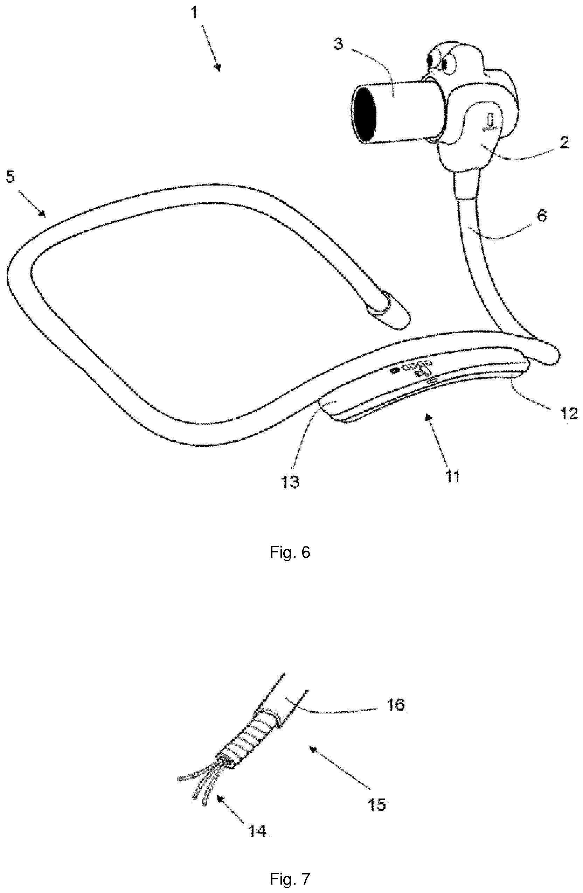

Figure 6 shows a perspective view of a sixth exemplary, non-limiting embodiment of the wearable spirometry arrangement 1 according to the invention wherein the adjustable support structure 5 is at least partially deformable and is provided with a control unit 13 that is accommodated in a support element 11 that has a soft pad 12 that in use can be arranged in abutting contact with a shoulder of the user. The spirometer 2 has a design that is appealing to children. This can increase their motivation to perform breathing exercises, lung function measurements or lung training.

Figure 7 shows a part of the adjustable support structure according to a first exemplary, non-limiting embodiment thereof. The part of the adjustable support structure shown in figure 7 comprises a flexible tubular element 15 that has an outer surface that is provided with one cover layer 16. In this way the flexible tubular element 15 can be protected from the environment in which the wearable spirometry arrangement 1 is used. The cover layer 16 can be a resilient layer in order to improve user comfort while wearing the spirometry arrangement. The cover layer 16 can be a solid foam layer. However, the skilled person will appreciate that any other material that is suitable to improve user comfort can be used. Furthermore, the skilled person will appreciate that depending on the requirements of a specific application of the wearable spirometry arrangement according to the invention, also multiple coaxially arranged cover layers can be applied. In such a case, at least the outermost cover layer preferably is a resilient layer.

The part of the flexible tubular element shown in figure 7 can be deformed to obtain a shape that conforms with the individual anatomical shape of at least a part of the torso of the user with which it can be placed in an abutting contact. The skilled person will appreciate that the flexible tubular element could be implemented by any construction that is suitable for achieving the aforementioned effect, for example a wire wound structure as shown in figure 7.

Furthermore, figure 7 shows that electrical wiring 14 is arranged in an inner space of the flexible tubular element 15. The electrical wiring 14 can be used to interconnect the spirometer and the control unit of the wearable spirometry arrangement to enable communication between them. The electrical wiring 14 can also be used to provide power to the spirometer and the control unit.

The present invention can be summarized as relating to a wearable spirometry arrangement 1 comprising a spirometer 2 having an inlet 3 for receiving a volume of air inspired and expired by a user 4, and an adjustable support structure 5 having a first end part 6 that is connected with the spirometer, said support structure being configurable to have at least two contact areas with at least one of a torso 10 and a neck 17 of the user, wherein a first contact area 7 of the at least two contact areas is arrangeable on a side of at least one of the torso and the neck of the user that is opposite to a side of at least one of the torso and the neck of the user on which a second contact area 8 of the at least two contact areas is arrangeable.

It will be clear to a person skilled in the art that the scope of the present invention is not limited to the examples discussed in the foregoing but that several amendments and modifications thereof are possible without deviating from the scope of the present invention as defined by the attached claims. While the present invention has been illustrated and described in detail in the figures and the description, such illustration and description are to be considered illustrative or exemplary only, and not restrictive.

The present invention is not limited to the disclosed embodiments. Variations to the disclosed embodiments can be understood and effected by a person skilled in the art in practicing the claimed invention, from a study of the figures, the description and the attached claims. In the claims, the word "comprising" does not exclude other steps or elements, and the indefinite article "a" or "an" does not exclude a plurality. The mere fact that certain measures are recited in mutually different dependent claims does not indicate that a combination of these measures cannot be used to advantage. Any reference numerals in the claims should not be construed as limiting the scope of the present invention.

### REFERENCE NUMERALS

- 1: wearable spirometry arrangement
- 2: spirometer
- 3: inlet
- 4: user
- 5: support structure
- 6: first end part of the support structure
- 7: first contact area
- 8: second contact area
- 9: third contact area
- 10: torso of the user
- 11: support element
- 12: soft pad
- 13: control unit
- 14: electrical wiring
- 15: flexible tubular element
- 16: cover layer
- 17: neck of the user

## Claims

1. A wearable spirometry arrangement (1) comprising:
- a spirometer (2) having an inlet (3) for receiving a volume of air inspired and expired by a user (4); and
- an adjustable support structure (5) having a first end part (6) that is connected with the spirometer, the adjustable support structure being configurable to have at least two contact areas with at least one of a torso (10) and a neck (17) of the user, wherein a first contact area (7) of the at least two contact areas is arrangeable on a side of at least one of the torso and the neck of the user that is opposite to a side of at least one of the torso and the neck of the user on which a second contact area (8) of the at least two contact areas is arrangeable.

2. The wearable spirometry arrangement (1) according to claim 1, wherein the adjustable support structure (5) is at least partially deformable.

3. The wearable spirometry arrangement (1) according to claim 1 or 2, wherein the adjustable support structure (5) is configurable at least over one shoulder of the user.

4. The wearable spirometry arrangement (1) according to any one of the preceding claims, wherein the adjustable support structure (5) is provided with at least one support element (11).

5. The wearable spirometry arrangement (1) according to claim 4, wherein the at least one support element (11) has an anatomical shape.

6. The wearable spirometry arrangement (1) according to claim 4 or 5, wherein the at least one support element (11) comprises at least one soft pad (12) that is arrangeable in abutting contact with at least one of the torso and the neck of the user.

7. The wearable spirometry arrangement (1) according to any one of the preceding claims, wherein the adjustable support structure (5) is provided with a control unit (13) that is configured and arranged to communicate with the spirometer (2).

8. The wearable spirometry arrangement (1) according to claim 7, wherein the control unit (13) is accommodated within the at least one support element (11).

9. The wearable spirometry arrangement (1) according to claim 7 or 8, wherein at least one of the spirometer (2) and the control unit (13) is configured and arranged to communicate with a remote system.

10. The wearable spirometry arrangement (1) according to any one of claims 7 to 9, wherein the adjustable support structure (5) comprises electrical wiring (14) that is arranged between the spirometer (2) and the control unit (13).

11. The wearable spirometry arrangement (1) according to any one of the preceding claims, wherein the adjustable support structure (5) comprises at least one flexible tubular element (15).

12. The wearable spirometry arrangement (1) according to claim 11, wherein the adjustable support structure (5) further comprises at least one rigid tubular element that is connected with the at least one flexible tubular element, wherein the at least one flexible tubular element (15) or the at least one rigid tubular element is connected with the spirometer (2).

13. The wearable spirometry arrangement (1) according to claim 12, wherein the adjustable support structure (5) comprises multiple flexible tubular elements and multiple rigid tubular elements that are alternatingly arranged.

14. The wearable spirometry arrangement (1) according to any one of the preceding claims, wherein the adjustable support structure (5) has an outer surface that is provided with at least one cover layer (16).

15. The wearable spirometry arrangement (1) according to claim 14, wherein the at least one cover layer (16) is a resilient layer.
